Europäisches Patentamt

(19) European Patent Office

Office européen des brevets

(11) Numéro de publication: **0 186 232**
**B1**

(12) FASCICULE DE BREVET EUROPEEN

(45) Date de publication du fascicule du brevet: **16.05.90**

(51) Int. Cl.⁵: **A 61 M 5/315**

(21) Numéro de dépôt: **85201992.6**

(22) Date de dépôt: **27.11.85**

(54) Seringue.

(30) Priorité: **07.12.84 CH 5832/84**
**11.01.85 CH 123/85**

(43) Date de publication de la demande:
**02.07.86 Bulletin 86/27**

(45) Mention de la délivrance du brevet:
**16.05.90 Bulletin 90/20**

(84) Etats contractants désignés:
**AT BE DE FR GB IT NL SE**

(56) Documents cités:
**FR-A-2 347 058**
**US-A-3 325 061**
**US-A-3 786 811**
**US-A-4 263 911**
**US-A-4 484 915**

(73) Titulaire: **Schweblin, Jean-Denis**
**4, place de l'Etrier**
**CH-1224 Chene-Bougeries (CH)**

(72) Inventeur: **Schweblin, Jean-Denis**
**4, place de l'Etrier**
**CH-1224 Chene-Bougeries (CH)**

(74) Mandataire: **Werner, Guy et al**
**Bugnion S.A. Case postale 375 10 Route de**
**Florissant**
**CH-1211 Genève 12 - Champel (CH)**

## Description

La présente invention a pour objet une seringue de prélèvement et de vidange d'un liquide, selon le préambule de la revendication 1.

Les seringues connues jusqu'ici, utilisées pour le prélèvement et la vidange de liquides, son du type où le piston amovible dans le corps cylindrique est retiré de celui-ci par l'entremise de la tige de piston, l'action sur la seringue étant celle d'écarter le corps, d'un côté, et la tige solidaire du piston, de l'autre. Ce type de seringue, qui existe aussi bien sous des formes réutilisables après stérilisation que sous des formes à utilisation unique où la seringue est jetée après usage, présente trois inconvénients majeurs. En premier lieu, il nécessite l'utilisation des deux mains pour le prélèvement, l'une tenant le corps de la seringue et l'autre la partie antérieure du piston. Au fur et à mesure que s'effectue le mouvement de prélèvement, les mains de l'utilisateur s'écartent et leur coordination devient moins précise. En deuxième lieu, le positionnement de la seringue sur le corps à travers lequel s'effectue le prélèvement est rendu difficile, parce que la main que tient le corps de la seringue représente un encombrement par rapport au corps du patient.

Enfin, en troisième lieu, l'opération de ponction et de prélèvement de la seringue doit se faire en deux temps, avec changement de la position des mains. Dans le premier temps, une main tient le corps du patient et l'autre main tient le seringue, pour introduire l'aiguille dans ce corps. Dans le deuxième temps, la main qui tient le corps du patient vient saisir le corps de la seringue, et celle qui tenait ce dernier vient saisir la tête de la tige du piston pour extraire ce dernier dans la fonction de prélèvement. Les deux mains sont alors mobilisées par la seringue.

Pour pallier la difficulté d'utiliseur les seringues connues d'une seule main, notamment dans leur fonction de prélèvement, on a déjà eu l'idée de munir l'extrémité antérieure de la tige du piston d'un anneau dans lequel le pouce du praticien peut se glisser. Toutefois, la force nécessaire au prélèvement, y compris la résistance de friction exercée par le piston sur le corps de seringue, sont souvent telles que le pouce ne parvient pas à effectuer l'opération ou que la tension qui en résulte est dangereuse pour les mouvements qu'elle peut donner à la seringue, avec pour conséquence un manque de précision dans la manoeuvre.

D'autres solutions ont été proposées pour faciliter l'opération de prélèvement et, bien que dans une moindre mesure, de vidange ou d'injection des seringues. On constate que ces solutions compliquent considérablement la construction de la seringue et la rendent coûteuse, ou modifient sérieusement les habitudes du praticien, qui peut alors être amené à des erreurs de manipulation.

Les recherches effectuées parmi l'art antérieur ont également permis de relever des constructions telles que décrites dans les brevets US N° 4 263 911, 3 325 061 et 4 484 915, où l'idée de prélever un liquide en rapprochant deux points d'appui reliés respectivement au corps et au piston d'une seringue est développée d'une façon relativement compliquée. Dans le premier brevet cité, toutefois, la manipulation de la seringue exige les deux mains de l'opérateur.

Dans les deuxième et troisième brevets mentionnés, les organes d'actionnement proposés sont extérieurs et accessoires à une seringue de type habituel et destinés à venir la compléter. Le nombre de pièces requis qui est d'au moins trois, augmente le coût de la construction de la seringue complète.

L'axe le long duquel s'exerce la pression est sensiblement oblique par rapport à l'axe de la seringue. La rapidité et la stabilité des parties coulissantes est obtenue au prix d'une augmentation du poids de la seringue complète et d'une complication des ajustements des parties entre elles.

La présente invention a pour objet une seringue obviant à ces inconvénients, notamment dans sa fonction de prélèvement, mais également dans sa fonction de vidange ou d'injection, d'une construction extrêment simple, d'une grande précision d'utilisation et ne modifiant pas sensiblement les habitudes acquises avec les seringues conventionnelles. Sa manutention peut s'effectuer d'une seule main, sans changement de position entre l'acte de ponction, la fonction de prélèvement et celle de vidange, cas échéant d'injection et ceci sans effort particulier pour les doigts de la main concernée.

La seringue selon l'invention est caractérisée par le fait que la tige de piston intérieure ainsi que la partie extérieure ont toutes les deux une section approximativement semi-circulaire et coiffent ledit corps cylindrique jusque dans la région du piston, que ladite partie terminale se prolonge vers l'extrémité postérieure dudit corps et forme ladite partie extérieure, qui coiffe la tige de piston intérieure et est reliée à celle-ci, le tout de manière qu'en rapprochant la troisième surface d'appui de la première, le volume de la chambre augmente, tandis qu'en éloignant la troisième surface d'appui de la première, le volume de la chambre diminue, et par le fait que la première surface d'appui présente un prolongement intérieur, de manière que le point d'application du pouce de la main de l'utilisateur sur ce prolongement se rapproche ou coïncide avec l'axe de déplacement du piston.

Le dessin annexé représente, à titre d'exemple, trois formes d'exécution de l'invention.

La figure 1 montre la première forme d'exécution, en perspective.

La figure 2 est une coupe longitudinale, selon l'axe II—II de la figure 1.

La figure 3 représente la deuxième forme d'exécution, en perspective.

La figure 4 est une coupe longitudinale selon l'axe IV—IV de la figure 3.

La figure 5 est une vue en perspective de la seringue selon la première forme d'exécution, actionnée pour un prélèvement.

La figure 7 montre la troisième forme d'exécution, en perspective.

La figure 8 est une vue en perspective de la seringue selon la troisième forme d'exécution, actionnée pour un prélèvement.

La figure 9 est une vue, semblable à celle de la figure 8, de la seringue actionnée pour une vidange.

Dans la description qui suit, les parties de la seringue du côté de l'embout porte-aiguille identifié ci-après sont appelées "postérieures". Celles qui sont de l'autre côté, vers l'extrémité de la tige de piston identifiée ci-après, sont appelées "antérieures".

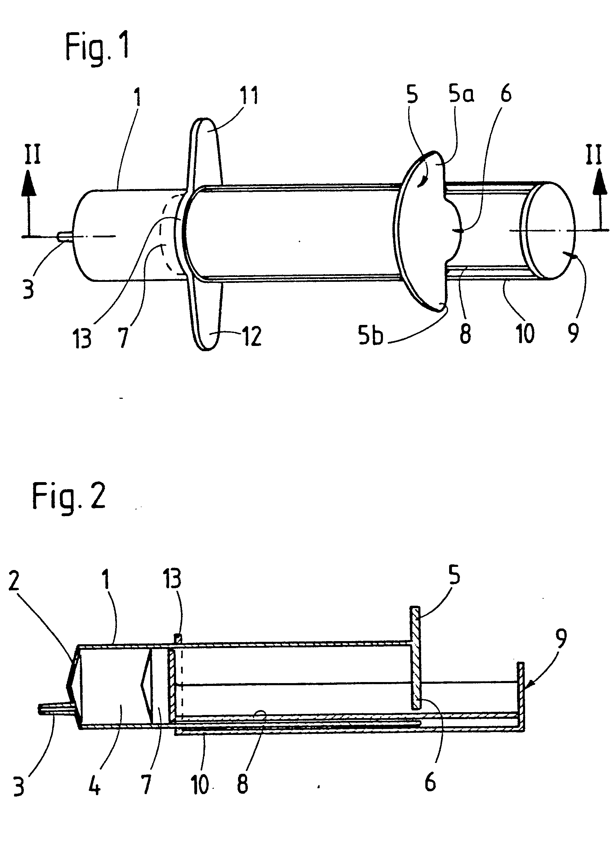

En se rapportant aux figures 1 et 2 on voit que la seringue se compose d'un corps cylindrique 1, dont l'extrémité postérieure 2 est obturée et porte un embout porte-aiguille 3 percé d'un canal non représenté donnant accès à la chambre 4. L'extrémité opposée du corps cylindrique 1 est partiellement recouverte d'une première surface d'appui formée d'une plaquette 5 en demi-lune dont le diamètre est supérieur à celui du corps 1 et présentant de préférence vers l'intérieur un prolongement semi-circulaire 6 de diamètre inférieur.

Un piston 7 de type habituel coulisse dans le corps cylindrique 1 et délimite le volume variable de la chambre 4. Ce piston est solidaire d'une tige de piston présentant une partie 8, intérieure et rigide, dont la section est une portion de cylindre de diamètre légèrement inférieur au diamètre intérieur du corps 1.

A son extrémité antérieure, cette tige 8 est coiffée par une plaquette circulaire, formant la troisième surface d'appui 9, de diamètre légèrement supérieur au diamétre extérieur du corps 1 et se prolongeant vers la partie postérieure dudit corps 1 par une seconde partie semi-circulaire 10, extérieure et coiffant le corps 1 jusque dans la région du piston 7.

Cette seconde partie semi-cylindrique extérieure 10 porte à sont extrémité postérieure deux ailettes radiales 11 et 12, sensiblement parallèles aux côtés 5a et 5b de la plaquette 5 et formant la deuxième surface d'appui.

La tige du piston est donc formée des parties 8, 9 et 10, enveloppant et coiffant le manteau du corps de cylindre 1.

Prolongeant ces ailettes, une portion de bague 13 retient la seconde partie semi-cylindrique 10 sur le corps 1. L'ensemble 7, 8, 9, 10, 11, 12 et 13, une fois monté, forme une seule pièce rigide.

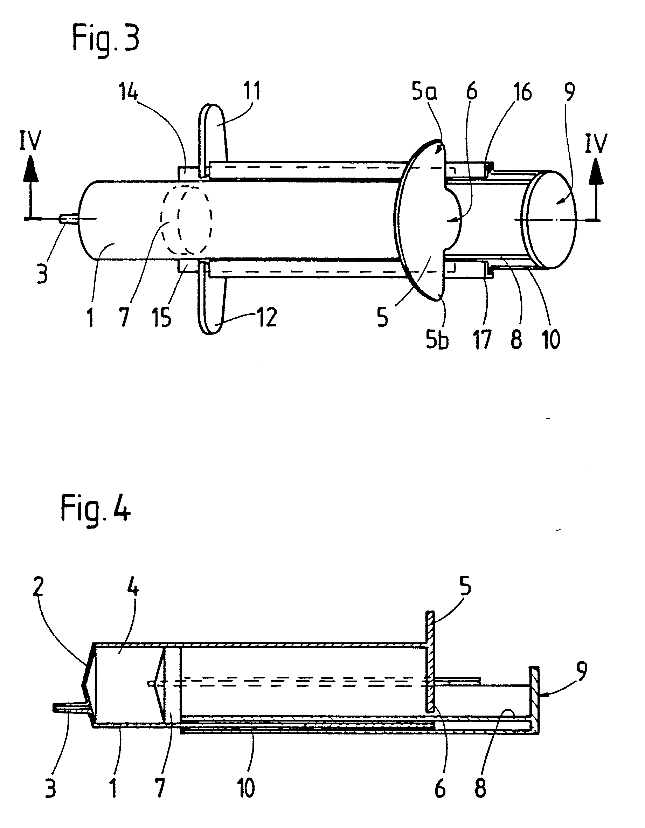

Dans la variante représentée aux figures 3 et 4 la bague 13, qui ne peut dans le cas de la première variante être fixée qu'après insertion du piston dans le corps 1, est remplacée par un système de guidage coulissant.

Celui-ci est constitué par deux épaulements longitudinaux 14 et 15 diamétralement opposés, venus de fabrication sur la partie cylindrique extérieure du corps 1 et par deux rabattements longitudinaux 16 et 17 en forme de U s'ouvrant vers l'intérieur, dans lesquels lesdits épaulements viennent coulisser. Dans ce cas, l'ensemble piston 7, 8, 9, 10, 11, 12, 16, 17 peut être monté amoviblement dans et sur le corps cylindrique 1 portant les épaulements de guidage et de retenue 14 et 15, si le prolongement semi-circulaire 6 est fixé après ou supprimé.

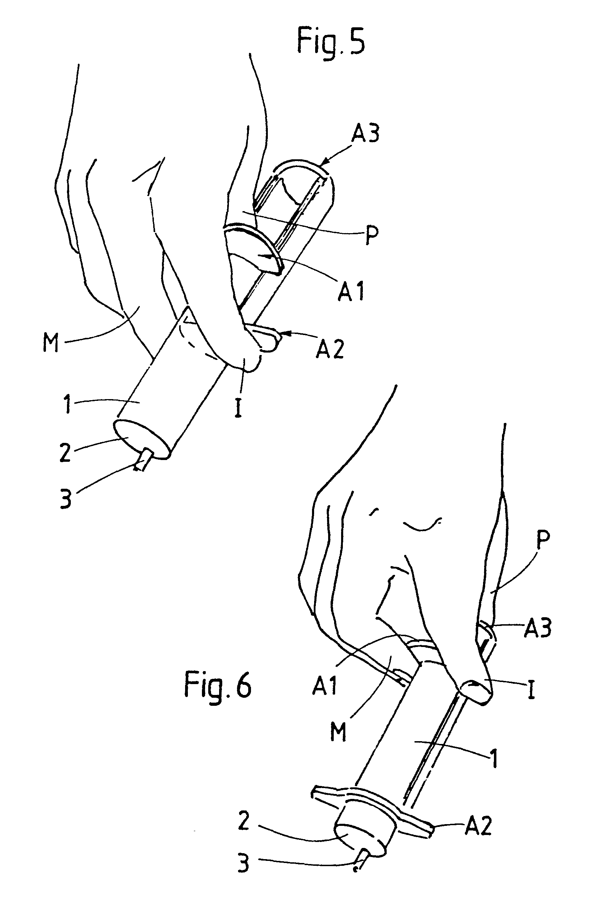

Pour simplifier l'explication du fonctionnement de la seringue dans l'une ou l'autre variante, on se reportera aux figures 5 et 6 où la première surface d'appui 5 est dénommée A1, la deuxième surface d'appui A2 et la troisième surface d'appui A3. Dans l'exemple représenté, l'opérateur utilise sa main droite, dont le pouce est désigné par P, l'index par I et le majeur par M.

En figure 5, la seringue est représentée en fonction de prélèvement l'index I et le majeur M s'appuyent sur la face postérieure des ailettes de la deuxième surface d'appui A2. Le pouce P s'appuie sur la face antérieure de la première surface d'appui A1. En rapprochant l'ensemble index et majeur du pouce, le piston 7 (voir figures 1 ou 3) s'éloigne de l'extrémité obturée 2 du corps 1 et le volume de la chambre intérieure 4 (voir figures 2 ou 4) augmente en aspirant du liquide. Cette opération, qui suit une ponction normale à l'aide d'une aiguille dans un corps non représenté, s'appelle le prélèvement.

La figure 6 montre l'opération de vidange (qui pourrait également être une opération d'injection). Ici, l'opérateur a simplement fait passer son pouce P de la première surface d'appui A1 à la troisième surface d'appui A3, puis son ensemble index I et majeur M de la deuxième surface d'appui A2 à la première surface d'appui A1. Le pouce, en se rapprochant de l'ensemble index et majeur, fait entrer le piston dans le corps et réduit le volume de la chambre par éjection du liquide vers l'extérieur, à travers l'embout, par exemple dans un récipient où le liquide est conservé.

On remarquera que pour passer de l'opération de prélèvement à l'opération de vidange l'autre main de l'opérateur ou du praticien n'a pas été mise à contribution.

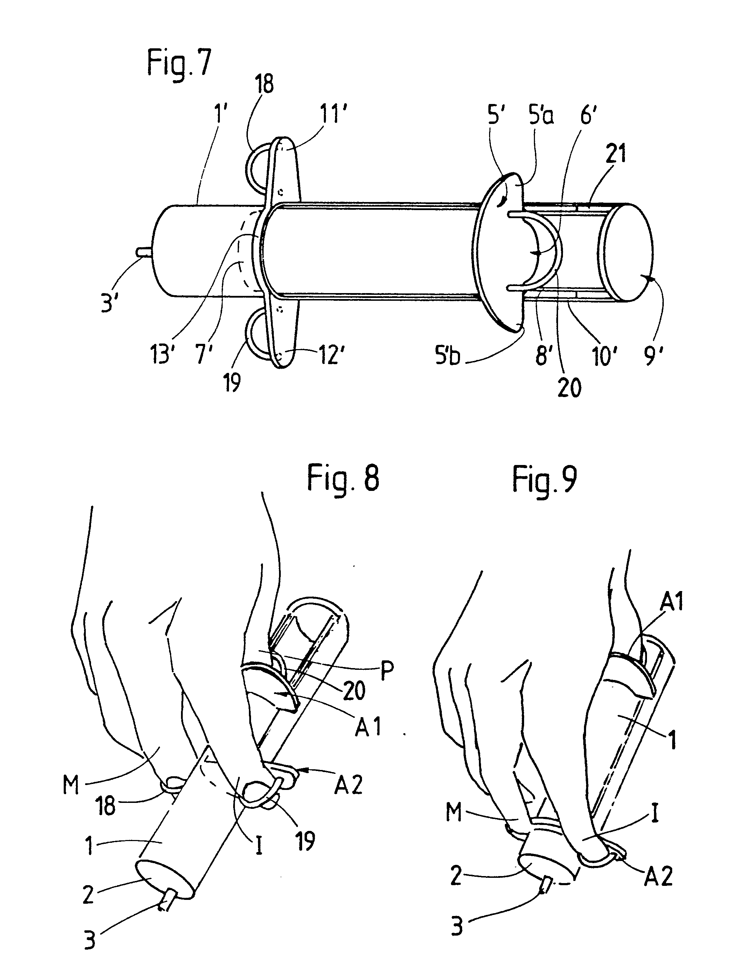

En se reportant maintenant à la figure 7 on comprendra en quoi résident les différences de la troisième forme d'exécution. Ici, les références identiques à celles de la figure 2 sont conservées avec l'indication prime (').

Sur la partie postérieure des ailettes 11' et 12' sont venues de fabrication des boucles 18 et 19. Alternativement l'ensemble ailette-boucle pourrait être remplacée par un anneau. Sur la partie antérieure de la première surface d'appui 5' est venue de fabrication une boucle 20. Sur cette boucle pourrait être alternativement monté un anneau. Destinées respectivement au pouce (20) et aux index et medium (19 et 18) ces boucles pourraient se présenter aussi sous forme d'autres types de logements permettant un mouvement alternatif des doigts dans le sens d'un rapprochement ou d'un éloignement, comme il est décrit à l'aide des figures suivantes, où n'interviennent pour le prélèvement et pour la vidange que les première et deuxième surfaces d'appui A1 et A2, pour augmenter la rigidité des parties 8' et 10' de la tige de piston, relevées par la plaque terminale 9' on peut prévoir une soudure 21 ne gênant pas la course de l'ensemble 5'—6'—20.

Dans la fonction de prélèvement (figure 8), l'utilisation de la seringue est la même que celle décrite pour la figure 5. Toutefois P est passé dans la boucle 20 et le medium et l'index M et I sont passés dans les boucles 18 et 19 respectivement.

La figure 9 montre l'opération de vidange. Ici, l'opération de vidange. Ici, l'opérateur ne modifie pas la position de ses doigts, comme c'était le cas dans la procédure décrite pour la figure 6. Simplement, après avoir rapproché l'index et le majeur du pouce pour le prélèvement (figure 8), il écarte ceux-ci du pouce, les uns et l'autre étant retenus par les boucles 18, 19 et 20. Lorsque le piston est bien lubrifié, cétte opération d'écartement des doigts est aisée.

La seringue proposée présente plusieurs avantages importants qu'il est utile de rappeler et d'expliciter. Tout d'abord, dans sa version habituelle en utilisation unique (jetée après usage) elle peut être fabriquée aussi simplement et économiquement que les seringues à usage unique que l'on connaît déjà.

Au moment de la ponction (introduction de l'aiguille dans le corps du patient), le groupe index-medium de la main du practicien est très proche de l'aiguille sans gêner la manoeuvre et permet une excellente précision de mouvement.

Dès la ponction faite, l'aspiration pour le prélèvement s'effectue, avec une précision et un doigté maximums, tout en permettant une correction ou une modification de la ponction elle-même. En effet, le guidage et l'aspiration peuvent se faire simultanément d'une seule main.

Le système de coulissage corps cylindrique-piston rend impossible tout blocage du piston dans le corps cylindrique. Par ailleurs l'actionnement de la seringue d'une seule main empêche le piston de sortir du corps cylindrique en fin de course.

Le coulissage corps cylindrique-piston évite un porte-à-faux préjudiciable à l'opération.

Enfin, et cela est extrêment important, le praticien ou l'opérateur conserve en permanence une main libre, puisque la seringue est actionnée d'une seule main dans toutes ses fonctions. La main libre est précieuse, par exemple vis-à-vis d'enfants ou de patients nerveux ou encore pour accompagner l'opération de prélèvement d'autres mouvements ou opérations utiles simultanément.

**Revendications**

1. Seringue de prélèvement et de vidange d'un liquide, comportant un corps cylindrique (1, 1') sur l'extrémité postérieure (2) obturée duquel est monté un embout porte-aiguilles (3, 3') et dont l'extrémité antérieure présente une première surface d'appui (5, 5'), un piston (7, 7') solidaire d'une tige de piston intérieure (8, 8'), mobile coaxialement dans ce corps (1, 1'), l'extrémité antérieure de cette tige intérieure (8, 8') se trouvant toujours en dehors dudit corps (1, 1'), une partie terminale (9, 9') étant prévue à cette extrémité antérieure utilisable comme une deuxième surface d'appui, et une partie extérieure (10, 10') entourant partiellement le manteau cylindrique dudit corps (1, 1') et s'étendant jusqu'à ladite partie temrinale (9, 9'), cette partie extérieure (10, 10') étant mobile, parallèle à l'axe de ce corps (1, 1') le long de son manteau, l'extrémité postérieure de cette partie extérieure portant une troisième surface d'appui (11, 12, 11', 12'), de telle manière qu'au mouvement relatif du corps (1, 1') et du piston (7, 7') corresponde une variation du volume de la chambre (4) comprise entre l'extrémité postérieure (2) du corps (1, 1'), son manteau cylindrique et la surface adjacente du piston, pour aspirer ou éjecter un liquide au travers de l'embout porte-aiguille (3, 3'), caractérisée par le fait que la tige de piston intérieure (8, 8') ainsi que la partie extérieure (10, 10') ont toutes les deux une section approximativement semi-circulaire et coiffent ledit corps cylindrique (1, 1') jusque dans la région du piston (7, 7'), que ladite partie terminale (9, 9') se prolonge vers l'extrémité postérieure dudit corps (1, 1') et forme ladite partie extérieure (10, 10'), qui coiffe la tige de piston intérieure (8, 8') et est reliée à celle-ci, le tout de manière qu'en rapprochant la troisième surface d'appui (11, 11', 12, 12') de la première (5, 5'), le volume de la chambre (4, 4') augmente, tandis qu'en éloignant la troisième surface (11, 12, 11', 12') d'appui de la première (5, 5') le volume de la chambre (4, 4') diminue, et par le fait que la première surface d'appui (5, 5') présente un prolongement intérieur (6, 6'), de manière que le point d'application du pouce de la main de l'utilisateur sur ce prolongement se rapproche ou coïncide avec l'axe de déplacement du piston.

2. Seringue selon la revendication 1, caractérisée par le fait que ledit prolongement intérieur (6, 6') a une forme semi-circulaire, de diamètre inférieur à celui du corps (1, 1').

3. Seringue selon la revendication 1 ou 2, caractérisée par le fait qu'une portion de bague (13, 13') ferme la partie extérieure (10, 10'), dans la région de la troisième surface (11, 12, 11', 12') d'appui, autour du corps cylindrique (1, 1'), de manière à permettre un coulissement axial de cette partie extérueure sur le corps en empêchant normalement leur séparation radiale.

4. Seringue selon l'une des revendications 1 à 3, caractérisée par deux épaulements longitudinaux, diamétralement opposés (14, 15) sur la périphérie extérieure du corps cylindrique (1, 1'), et par deux rabattements longitudinaux (16, 17) des arêtes de la partie extérieure (10, 10') venant s'accrocher de part et d'autre de ces épaulements, de manière à permettre un coulissement aixal de la partie extérieure sur le corps en empêchant normalement leur séparation radiale.

5. Seringue selon l'une des revendications 1 à 4, caractérisée par deux rainures longitudinales diamétralement opposées creusées dans la périphérie extérieure du corps cylindrique (1, 1') et par deux rabattements longitudinaux vers l'intérieur des arêtes de la partie extérieure (10, 10'), venant coulisser dans lesdites rainures en empêchant normalement la séparation radiale de la seconde partie extérieure et du corps.

6. Seringue selon l'une des revendications 1 à 5, caractérisée par le fait que la première surface d'appui (5') comporte sur sa partie antérieure un logement annulaire (20) et par le fait que la troisième surface d'appui (11', 12') est formée de deux ailettes latérales opposées, sur chacune des parties postérieures desquelles sont conformés deux logements annulaires (18, 19).

7. Seringue selon l'une des revendications 1 à 6, caractérisée par le fait que la troisième surface d'appui est formée de deux ailettes latérales opposées (11, 12, 11', 12').

## Patentansprüche

1. Spritze zur Entnahme und Entleerung einer Flüssigkeit mit einem zylindrischen Körper (1, 1'), an dessem hinteren verschlossenen Ende (2) ein Nadelhalter (3, 3') montiert ist und dessen vorderes Ende eine erste Anlagefläche (5, 5') aufweist, mit einem Kolben (7, 7'), der fest an einer inneren Kolbenstange (8, 8') angebracht und koaxial in diesem Körper (1, 1') beweglich ist, wobei sich das vordere Ende dieser inneren Kolbenstange (8, 8') stets ausserhalb des erwähnten Körpers (1, 1') befindet, mit einem an diesem vorderen Ende vorgesehenen Endteil (9, 9'), welcher als eine zweite Anlagefläche verwendbar ist, und mit einem äusseren Teil (10, 10'), welche teilweise den zylindrischen Mantel des erwähnten Körpers (1, 1') umgibt und sich bis zu dem erwähnten Endteil (9, 9') erstreckt, wobei dieser äussere Teil (10, 10') parallel zur Achse dieses Körpers (1, 1') längs seines Mantels beweglich ist und das hintere Ende dieses äusseren Teils eine dritte Anlagefläche (11, 12, 11', 12') derart trägt, dass einer Relativverschiebung des Körpers (1, 1') und des Kolbens (7, 7') eine Volumenänderung der Kammer (4) zwischen dem hinteren Ende (2) des Körpers (1, 1'), seinem zylindrischen Mantel und der benachbarten Fläche des Kolbens entspricht, um eine Flüssigkeit durch den Nadelhalter (3, 3') anzusaugen oder auszustossen, dadurch gekennzeichnet, dass die innere Kolbenstange (8, 8') und der äussere Teil (10, 10') beide einen näherungsweise halbkreisförmigen Querschnitt haben und den erwähnten zylindrischen Körper (1, 1') bis zum Bereich des Kolbens (7, 7') überdecken, dass der erwähnte Endteil (9, 9') bis zum hinteren Ende des erwähnten Körpers (1, 1') verlängert ist und den erwähnten äusseren Teil (10, 10') bildet, welcher die innere Kolbenstange (8, 8') überdeckt und mit dieser verbunden ist, derart, dass beim Annähern der dritten Anlagefläche (11, 11', 12, 12') an die erste Anlagefläche (5, 5') sich das Volumen der Kammer (5, 5') vergrössert, während beim Entfernen der dritten Anlagefläche (11, 12, 11', 12') von der ersten Anlagefläche (5, 5') sich das Volumen der Kammer (4, 4') verkleinert, und dass die erste Anlagefläche (5, 5') eine innere Verlängerung (6, 6') aufweist, derart, dass der Druckpunkt des Daumens der Hand des Benutzers in der Nähe der Verschiebungsachse des Kolbens liegt oder mit dieser zusammenfällt.

2. Spritze nach Anspruch 1, dadurch gekennzeichnet, dass die innere Verlängerung (6, 6') halbkreisförmig ist und einen Durchmesser hat, der kleiner als der des Körpers (1, 1') ist.

3. Spritze nach Anspruch 1 oder 2, dadurch gekennzeichnet, dass ein Teil eines Ringes (13, 13') den äusseren Teil (10, 10') im Bereich der dritten Anlagefläche (11, 12, 11', 12') um den zylindrischen Körper (1, 1') schliesst, derart, dass dieser äussere Teil axial auf dem Körper verschiebbar ist, eine radiale Trennung beider Teile jedoch normalerweise verhindert wird.

4. Spritze nach einem der Ansprüche 1 bis 3, dadurch gekennzeichnet, dass auf dem äusseren Umfang des zylindrischen Körpers (1, 1') zwei diametral gegenüberliegende, longitudinale Schultern (14, 15) vorgesehen sind und dass der äussere Teil (10, 10') zwei Kanten in Form longitudinaler Umbördelungen (16, 17) aufweist, welche diese Schultern beiderseits einschliessen, derart, dass der äussere Teil auf dem Körper verschiebbar ist, eine radiale Trennung dieser Teile jedoch normalerweise verhindert wird.

5. Spritze nach einem der Ansprüche 1 bis 4, dadurch gekennzeichnet, dass auf dem Aussenumfang des zylindrischen Körpers (1, 1') zwei diametral gegenüberliegende, longitudinale Nuten eingelassen sind und dass der äussere Teil (10, 10') zwei nach innen gerichtete Kanten in Form von longitudinalen Umbördelungen aufweist, welche in den erwähnten Nuten verschiebbar sind, eine radiale Trennung des zweiten äusseren Teils und des Körpers normalerweise jedoch verhindern.

6. Spritze nach einem der Ansprüche 1 bis 5, dadurch gekennzeichnet, dass die erste Anlagefläche (5') an ihrem vorderen Teil eine ringförmige Aufnahme (20) aufweist und dass die dritte Anlagefläche (11', 12') von zwei seitlich gegenüberliegenden Schenkeln gebildet wird, an deren hinteren Teilen zwei ringförmige Aufnahmen (18, 19) gebildet sind.

7. Spritze nach einem der Ansprüche 1 bis 6, dadurch gekennzeichnet, dass die dritte Anlagefläche von zwei seitlichen, gegenüberliegenden Schenkeln (11, 12, 11', 12') gebildet wird.

## Claims

1. Syringe for extracting and emptying a liquid comprising a cylindrical body (1, 1') provided on its close rear end (2) with a needle supporting nozzle (3, 3') and at its front end with a first bearing surface (5, 5') and a piston (7, 7') rigid with an inner piston rod (8, 8') and movable coaxially in said body (1, 1'), the front end of said inner rod (8, 8') being always outside of said body (1, 1'), said front end being provided with a terminal part (9, 9') for use as a second bearing surface, and an outer part (10, 10') enclosing partially the cylindrical wall of said body (1, 1') and extending to said terminal part (9, 9'), said outer part (10, 10') being movable, paralelly to the axis of said body (1, 1') along its wall, the rear end of fuid outer part being provided with a third bearing surface (11, 12, 11', 12') so that a relative movement between the body (1, 1') and the piston (7, 7') will correspond to a variation in the volume of the chamber (4) formed between the rear end (2) of the body (1, 1'), its cylindrical wall and the adjacent surface of the piston, for sucking or ejecting a liquid through said needle supporting nozzle (3, 3') characterized by the fact that the inner piston rod (8, 8') and the outer part (10, 10') are both of approximate semicircular cross section and enclose said cylindrical body (1, 1') up to the region of the piston (7, 7'), that said terminal part (9, 9') extends to the rear end of said body (1, 1') and forms said outer part (10, 10'), which encloses the inner piston rod (8, 8'), and is connected herewith, so that when the third bearing surface (11, 12, 11', 12') is moved toward the first bearing surface (5, 5') the volume of the chamber (4, 4') increases whereas when said third bearing surface (11, 12, 11', 12') is moved away from said first bearing surface (5, 5') the volume of said chamber decreases, and by the fact that said first bearing surface (5, 5') is provided with an inner extension (6, 6') so that the thumb of the operator's hand may bear on said surface along or substantially along the piston axis.

2. Syringe according to Claim 1, characterized by the fact that said inner extension (6, 6') is of semi-circular shape, its diameter being smaller than the diameter of the body (1, 1').

3. Syringe according to Claims 1 or 2, characterized by the fact that a ring segment (13, 13') closes the outer part (10, 10'), in the region of the third bearing surface (11, 12, 12', 12'), around the cylindrical body (1, 1') so that this outer part may slide along the body without risking to separate radially from it.

4. Syringe according to one of Claims 1 to 3, characterized by two diametrically opposed longitudinal shoulders (14, 15) on the outer periphery of the cylindrical body (1, 1') and by two longitudinal U-shaped portions (16, 17) of the edges of the outer part (10, 10'), engaging both sides of said shoulders, so that said outer part may slide axially relative to the body without risking to separate radially from it.

5. Syringe according to one of Claims 1 to 4, characterized by two diametrically opposed longitudinal grooves in the outer periphery of the cylindrical body (1, 1') and by two longitudinal portions folded into the inner of the edges of the outer part (10, 10') entering said grooves, so that this second outer part may slide along the body without risking to separate radially from it.

6. Syringe according to one of Claims 1 to 5, characterized by the fact that the first bearing surface (5') shows on its front end an annular recess (20), and by the fact that the third bearing surface (11', 12') is formed of two opposed laterally extending wings on the rear parts of which are provided two annular recesses (18, 19).

7. Syringe according to one of Claims 1 to 6, characterized by the fact that said third bearing surface is formed of two opposed laterally extending wings (11, 12, 11', 12').

Fig. 1

Fig. 2

## Fig. 3

## Fig. 4

Fig. 5

Fig. 6

Fig. 7

Fig. 8

Fig. 9